# EUROPEAN PATENT SPECIFICATION

(11) **EP 4 171 489 B1**
(45) Date of publication and mention of the grant of the patent: **25.03.2026**
(21) Application number: 21735325.9
(22) Date of filing: 28.06.2021
(51) Int. Cl.: A61K 8/41, A61Q 17/00, A01N 33/12, A61P 31/02, A61P 31/04, A61P 31/12, A61P 31/14

(54) **SANITIZING COMPOSITION**
DESINFEKTIONSZUSAMMENSETZUNG
COMPOSITION DE DÉSINFECTION

(30) Priority: 30.06.2020 IN 202021027813; 24.09.2020 EP 20198202
(43) Date of publication of application: 03.05.2023
(73) Proprietor: Unilever IP Holdings B.V., 6708 WH Wageningen (NL); Unilever Global IP Limited, Wirral, Merseyside CH62 4ZD (GB)
(72) Inventor: DASGUPTA, Anindya, Whitefield, Bangalore 560 066 (IN); GENCARELLI, Suzanne, Lynn, Trumbull, CT 06611 (US); KRISIAK, Jessica, Ann, Trumbull, CT 06611 (US); SAJI, Maya, Treesa, Whitefield, Bangalore 560 066 (IN); SALGAONKAR, Neha, Whitefield, Bangalore 560 066 (IN); SUBRAMANIAN, Vivek, Trumbull, CT 06611 (US); YOKUBINAS, Leonora, H., Trumbull, CT 06611 (US)
(74) Representative: Unilever Patent Group
(86) International application number: PCT/EP2021/067750
(87) International publication number: WO 2022/002882

(56) References cited:
- WO-A1-2020/109859
- CN-A- 109 125 122
- DE-A1- 3 339 196
- US-A1- 2010 278 906
- CRODA: "INCROQUAT(TM) BEHENYL TMS", CRODA INC, 11 December 2013 (2013-12-11), pages 1 - 5, XP055781960, Retrieved from the Internet <URL:https://www.ulprospector.com/documents/991883.pdf?bs=134&b=93026&st=20&r=na&ind=personalcare> [retrieved on 20210304]

## Description

### Field of the invention

The present invention is directed to compositions for use as a sanitizing composition. More particularly, the invention is directed to compositions for use as a sanitizing composition that comprise at least 80% by weight water, and a cationic surfactant. The composition for use according to the present invention when topically applied, unexpectedly, provides superior antimicrobial benefits while simultaneously delivering excellent antiviral activity. Such a composition results in a bacteria log kill of at least 2 and a viral log inactivation of at least 2 in less than 3 minutes after topical application, even when formulated with little to no ethanol. The sanitizing composition for use according to the present invention is particularly mild on skin.

### Background of the Invention

Consumer concern associated with microbial and viral contamination is usually high. This is true because the astute consumer realizes the negative impact such contaminates can have on his or her health. In fact, ailments such as sinus, ear and throat infections as well as bronchitis, pneumonia and whooping cough all link to bacteria as the culprit. Viruses, which are parasites, are typically linked to the common cold, the flu and certain pneumonias.

Consumers know it is generally good practice to regularly clean their hands and not touch their face in order to minimize the risk of getting sick. During epidemics, such as the one caused by ebola, and pandemics like those caused by influenza, cholera and HIV/AIDS, consumers are well aware that these serious infectious diseases are best defeated with regimens that include medical treatment, social distancing, the use of face masks and extensive hand washing. Information provided by the Centers for Disease Control and Prevention suggests that the COVID-19 (coronavirus) pandemic is a direct result of a virus that is more efficient and severe than influenza and that spreads rapidly from person to person via respiratory droplets. During such a pandemic, consumers know it is in their best interest to regularly wash and sanitize their hands.

Sanitizing products are commercially available and they are typically supplied as gels having at least 60% by weight or more of ethanol. While such products are known to yield good antibacterial benefits after application, certain consumers shun using high ethanol-based products since such products may dry the skin, weaken the epidermis barrier and expedite the aging process.

It is of increasing interest to develop a sanitizing composition that provides excellent antimicrobial and antiviral benefits while at the same time being safe and mild enough to use an unlimited amount of times throughout the day. Given consumer demands, it is of increasing interest to develop a sanitizing composition that also delivers soothing and moisturizing benefits when topically applied and that is gentle enough to use on the face and the most delicate consumer, babies. This invention, therefore, is directed to a composition for use as a sanitizing composition that comprises at least 80% by weight water, and a cationic surfactant. The composition for use according tof the present invention when opically applied, unexpectedly, provides superior antimicrobial benefits while simultaneously delivering excellent antiviral activity. Such a composition results in a bacteria log kill of at least 2 and a viral log inactivation of at least 2 in less than 3 minutes after topical application and even when formulated with little to no ethanol. The composition for use according to the present invention is also suitable to sanitize inanimate surfaces such as table tops, kitchen counters, clothing and the like.

### Additional Information

Efforts have been disclosed for making compositions that provide antimicrobial benefits. In U.S. patent nos. 4,374,126 and 5,417,968, compositions that impart antimicrobial benefits are described.

Other efforts have been disclosed for making compositions that provide antimicrobial benefits. In World Application WO08061375A1, hand sanitizers with a four carbon alkyl alcohol and lactic acid are described.

Still other efforts have been disclosed for making compositions that provide antimicrobial benefits. In U.S. patent no. 9,044,403, hand sanitizers with peracetic acid are described.

Even other efforts have been disclosed for making compositions that provide antimicrobial benefits. In U.S. patent application no. 2013202484, hand sanitizers with a chlorite solution are described.

US 2010/278906 A1 discloses an alcohol free antimicrobial composition to be used as skin disinfectant comprising over 4% by weight cationic surfactants and over 4% by weight cetyl alcohol.

None of the additional information describes a composition for use as a sanitizing composition as claimed in the present invention.

### Summary of the Invention

In a first aspect, the present invention is directed to the use of the composition as defined in claim 1.

In a second aspect, the present invention is directed to the composition for use as defined in claim 14.

All other aspects of the present invention will more readily become apparent from the description and examples which follow.

Skin, as used herein, is meant to include skin on the arms (including underarms), face, feet, neck, chest, hands, legs, buttocks and scalp (including hair). Sanitizing as used herein means a bacteria log kill of at least 2 and a viral log inactivation of at least 2 (both achieved) in less than 3 minutes after topical application to a surface. Sanitizing precursor means a composition which is a concentrate and suitable to be diluted (or hydrated) with water to produce a sanitizing composition ready for use. Combination, as used herein means, total weight of cetrimonium chloride plus benzalkonium chloride. Skin benefit agent means an ingredient suitable to improve a skin characteristic. Surface as used herein includes skin or the surface of an inanimate object such as a table top, computer monitor, door knob, toilet seat, shopping cart handle or even a clothing garment. Surface is also meant to include the coat of an animal such as the fur on a dog and cat. As used herein, surface preferably means human skin, and especially, skin on the face and hands. The dimonium and trimonium compounds used herein are meant to include salts of the same, especially chlorides and bromides of the same. In an embodiment of the invention, the sanitizing composition for use according tof the present invention is a home care composition like a laundry spray composition suitable to spray clothing and upholstery requiring sanitizing. The home care composition can also be a sanitizing kitchen or bathroom spray composition. In a preferred embodiment, the composition for use according to the present invention is a topical composition to apply to skin for sanitizing by significantly reducing the amount of bacteria and viruses on the skin. The composition for use according to the present invention may optionally comprise skin benefit agents added thereto such as emollients, vitamins and/or derivatives thereof, resorcinols, retinoic acid precursors, colorants, moisturizers or humectants, fragrances, sunscreens, a mixture thereof or the like. The skin benefit ingredients may be water or oil soluble. The sanitizing composition for use according to the invention, therefore, is an aqueous based composition with a pH from 3.0 to 8.2, and the composition is water continuous. Viscosity, as used herein, is taken with a Brookfield RV spindle 6 at 20 rpm for 30 seconds at 25°C unless noted otherwise. In still another embodiment, the composition for use according to this invention is a non-therapeutic and non-medicinal composition ( but including cosmetic in nature) which is a water continuous liquid having a viscosity under 30 Pa.s (30,000 cps). In the absence of explicitly stating otherwise, all ranges described herein are meant to include all ranges subsumed therein. As used herein, substantially free of means less than 10% by weight. Antimicrobial benefits mean at least a log kill of 2 in under 3 minutes whereby antimicrobial assessment is measured via test method ASTM-E2783-11 (Reapproved 2016) which sets forth the procedure for measuring antimicrobial activity for water miscible compounds using a time kill procedure. Viral (or virus) inactivation is determined by assessing the impact of microbiocides against viruses as set forth in ASTM- 1052-20. The term comprising is meant to encompass the terms consisting essentially of and consisting of. For the avoidance of doubt, and for illustration, a composition comprising water, cationic surfactant and preservative is meant to include a composition consisting essentially of the same and a composition consisting of the same. Except in the operating comparative examples, or where otherwise explicitly indicated, all numbers in this description indicating amounts or ratios of materials or conditions and/or physical properties of materials and/or use are to be understood as modified by the word "about".

### Detailed Description of the Preferred Embodiments

The trimonium compound used is an alkyl trimonium compound comprising cetrimonium chloride, cetrimonium bromide, mytrimonium chloride, mytrimonium bromide, behentrimonium methosulfate, cocotrimonium methosulfate, behentrimonium chloride, behentrimonium bromide, steartrimonium chloride, steartrimonium bromide, laurtrimonium chloride, laurtrimonium bromide, or a mixture thereof.. In an embodiment of the invention, the cationic surfactant used comprises cetrimonium chloride, cetrimonium bromide, mytrimonium chloride, mytrimonium bromide, behentrimonium methosulfate, cocotrimonium methosulfate, or a mixture thereof. In still another embodiment of the invention, the cationic surfactant comprises cetrimonium chloride, cetrimonium bromide or a mixture thereof. In yet another embodiment, the cationic surfactant comprises cetrimonium chloride. For the avoidance of doubt, cationic surfactant used in the present invention can consist essentially of or consist of any combination of the aforementioned surfactants.

The dimonium compound used is distearyl dimonium chloride, didecyl dimonium chloride, dicoco dimonium chloride, a mixture thereof or benzethonium chloride and/or benzalkonium chloride.

The cationic surfactant used in the composition for use according to the invention is 95 to 100% by weight trimonium compound (as defined above), dimonium compound (as defined above) or both. In an embodiment of the invention, the cationic surfactant is 98 to 100% by weight trimonium compound, preferably 100% by weight trimonium compound. In a preferred embodiment, the cationic surfactant used in the composition for use in the present invention is 90 to 100% by weight cetrimonium chloride, and more preferably, 98 to100% by weight cetrimonium chloride, and most preferably,100% by weight cetrimonium chloride.

As previously noted, when total surfactant in the composition for use in the invention is more than 4.5% by weight and cationic surfactant in the composition includes a combination of both cetrimonium chloride and benzalkonium chloride, benzalkonium chloride makes up at least 16.5% by weight of the total weight of the combination. In an embodiment of the invention, when total surfactant in the composition for use in the invention is more than 5% by weight and cationic surfactant in the composition includes a combination of both cetrimonium chloride and benzalkonium chloride, benzalkonium chloride makes up at least 16.5% by weight of the combination, and preferably, at least 18%, and most preferably, 20 to 60% (even more preferably, 30 to 55%) by weight of the combination. In even another embodiment, when total surfactant in the composition for use in the invention is more than 5.65% (preferably more than 6%) by weight and cationic surfactant in the composition includes a combination of both cetrimonium chloride and benzalkonium chloride, benzalkonium chloride makes up at least 16.5% by weight of the combination, and preferably, at least 18% by weight of the combination and most preferably, 20 to 30% by weight of the combination.

Cationic surfactant makes up from 0.08 to 0.4% by weight of the composition for use according to the invention.

When both trimonium and dimonium surfactant are used they are often used in a weight ratio of 1:99 to 99:1, and preferably, from 15:85 to 85:15, and most preferably, from 30:70 to 70:30, with the proviso that when total surfactant in the composition for use in the invention is more than 4.5% by weight and cationic surfactant in the composition includes a combination of both cetrimonium chloride and benzalkonium chloride, benzalkonium chloride makes up at least 16.5% by weight of the combination.

In another embodiment of the invention, the cationic surfactant used in the composition for use according to the present invention is at least 70%, and preferably, at least 80%, and most preferably, at least 90% by weight trimonium compound (as defined above). In even another embodiment of the invention, from 92 to 100% by weight of all cationic surfactant in the composition for use in the invention is trimonium compound. In still another embodiment, from 95 to 100% by weight of all cationic surfactant is trimonium compound. In yet another embodiment, 100% by weight of the cationic surfactant present in the composition for use according to the present invention is trimonium compound.

The composition for use according to the present invention has less than 0.3%, and preferably, less than 0.2%, by weight benzalkonium chloride and/or benzethonium chloride, and preferably, no (0.0% by weight) benzalkonium chloride and/or benzethonium chloride.

In still another embodiment of the invention, dimonium and/or trimonium compound (most preferably cetrimonium chloride) makes up at least 4%, and preferably, at least 4.5%, and most preferably, at least 5% by weight of the total weight of the surfactant in the sanitizing composition for use according to the invention.

Water makes up at least 80% by weight of the composition, and preferably, at least 82% by weight of the composition, and most preferably, at least 84% by weight of the sanitizing composition for use according to the invention. In an embodiment of the invention, water makes up from 85 to 99.5%, and preferably, from 87 to 98.8%, and most preferably, from 89 to 97% by weight of the composition for use according to the invention, including all ranges subsumed therein.

The sanitizing composition for use according to the present invention has less than 10% by weight ethanol, and preferably, comprises less than 6% by weight ethanol, and most preferably, from 2.5 to 5.5% by weight ethanol. In an embodiment of the invention, the composition for use according to the present invention may have less than 1% by weight ethanol. In another desired embodiment, the composition for use according to the invention may have no (0.0% by weight) ethanol. Additionally, and excluding ethanol and not including diols and polyols, the composition for use according to the present invention comprises no (0.0% by weight) aliphatic alcohol.

It is within the scope of this invention to include optional surfactants along with the cationic surfactants described herein. The optional surfactants can include those which are amphoteric (which depending on pH can be zwitterionic). Illustrative optional amphoteric surfactants suitable for use generally include sodium acyl amphoacetates, sodium acyl amphopropionates, disodium acyl amphodiacetates and disodium acyl amphodipropionates where the acyl (i.e., alkanoyl group) can comprise a C₇-C₁₈ alkyl portion. Illustrative examples of the amphoteric surfactants that may be used include sodium lauroamphoacetate, sodium cocoamphoacetate, sodium lauroamphoacetate, sodium cocoamphoacetate a mixture thereof or the like.

As to zwitterionic surfactants that may optionally be used in the sanitizing composition for use according to the present invention, such surfactants typically include at least one acid group. Such an acid group may be a carboxylic or a sulphonic acid group. They often include quaternary nitrogen, and therefore, can be quaternary amino acids. They should generally include an alkyl or alkenyl group of 7 to 18 carbon atoms and generally comply with an overall structural formula:

R1--[--C(O)--NH(CH₂)_{q}--]ᵣ--N⁺(R²)(R³)A--B

where R¹ is alkyl or alkenyl of 7 to 18 carbon atoms; R² and R³ are each independently alkyl, hydroxyalkyl or carboxyalkyl of 1 to 3 carbon atoms; q is 2 to 4; r is 0 to 1; A is alkylene of 1 to 3 carbon atoms optionally substituted with hydroxyl, and B is --CO₂-- or --SO₃--.

Suitable zwitterionic surfactants for use in the composition for use according to the present invention and within the above general formula include simple betaines of formula:

R¹--N⁺(R²)(R³)-CH₂CO₂⁻

and amido betaines of formula:

R¹--CONH(CH₂)ₜ--N⁺--(R²)(R³)CH₂CO₂⁻

where t is 2 or 3.

In both formulae R¹, R² and R³ are as defined previously. R¹ may, in particular, be a mixture of C₁₂ and C₁₄ alkyl groups derived from coconut oil so that at least half, preferably at least three quarters of the groups R¹ have 10 to 14 carbon atoms. R² and R³ are preferably methyl.

A further possibility is that the zwitterionic surfactant is a sulphobetaine of formula:

R¹--N⁺(R²)(R³)-(CH₂)₃SO₃⁻

or

R¹--CONH(CH₂)ᵤ --N⁺(R²)(R³)-(CH₂)₃SO₃⁻

where u is 2 or 3, or variants of these in which --(CH₂)₃SO₃⁻ is replaced by-CH₂C(OH)(H)CH₂SO₃⁻.

In these formulae, R¹, R² and R³ are as previously defined.

Illustrative examples of the zwitterionic surfactants suitable for optional use include betaines like cocodimethyl carboxymethyl betaine, cocoamidopropyl betaine and laurylamidopropyl betaine. An additional zwitterionic surfactant suitable for use includes cocoamidopropyl sultaine. Such surfactants are made commercially available from suppliers like Stepan Company, and it is within the scope of the invention to optionally employ mixtures of the same.

Nonionic surfactants that may optionally be used in the sanitizing composition for use according to the present invention include in particular the reaction products of compounds having a hydrophobic group and a reactive hydrogen atom, for example aliphatic alcohols, acids, amides or alkylphenols with alkylene oxides, especially ethylene oxide either alone or with propylene oxide. Specific nonionic surfactant compounds are alkyl (C₆-C₂₂) phenols ethylene oxide condensates, the condensation products of aliphatic (C₈-C₁₈) primary or secondary linear or branched alcohols with ethylene oxide, and products made by condensation of ethylene oxide with the reaction products of propylene oxide and ethylenediamine. Other nonionic surfactants include long chain tertiary amine oxides, long chain tertiary phosphine oxides, dialkyl sulphoxides or the like.

In an embodiment of the invention, optional nonionic surfactants include fatty acid/alcohol ethoxylates having the following structures a) HOCH₂(CH₂)ₛ(CH₂CH₂O)ᵥ H or b) HOOC(CH₂)_{c}(CH₂CH₂O)_{d} H; where s and v are each independently an integer up to18; and c and d are each independently an integer from 1 or greater. In an embodiment of the invention, s and v are each independently 6 to 18; c and d are each independently 1 to 30. Other options for nonionic surfactants include those having the formula HOOC(CH₂)ᵢ-CH=CH--(CH₂)ₖ(CH₂CH₂O)_{z} H, where i, k are each independently 5 to 15; and z is 5 to 50. In another embodiment of the invention, i and k are each independently 6 to 12; and z is 15 to 35.

The optional nonionic may also include a sugar amide, such as a polysaccharide amide. Specifically, the surfactant may be one of the lactobionamides described in U.S. Pat. No. 5,389,279 to Au et al., entitled "Compositions Comprising Nonionic Glycolipid Surfactants issued Feb. 14, 1995; or it may be one of the sugar amides described in U.S. Pat. No. 5,009,814 to Kelkenberg, titled "Use of N-Poly Hydroxyalkyl Fatty Acid Amides as Thickening Agents for Liquid Aqueous Surfactant Systems" issued Apr. 23, 1991;

Yet other optional nonionic surfactants that may be used in the composition for use according to the present invention include nonionic glucamides like lauroyl methyl glucamide, myristoyl methyl glucamide, cocoyl methyl glucamide, capryloyl/caproyl methyl glucamide, sunfloweroyl methyl glucamide mixtures thereof or the like.

Even other optional nonionic surfactants suitable for use include polyglycerol esters like polyglycerol-10 caprylate, polyglycerol-10 caprate, polyglycerol-10 laurate, polyglycerol-10 myristate, polyglycerol-10 palmitate, polyglycerol-10 stearate, polyglycerol-10 oleate, polyglyceryl-10 cocoate or a mixture thereof.

In still another embodiment of the invention, nonionic surfactant optionally used can include surfactants formed by the ethoxylation of sorbitan before the addition of a fatty acid. These include polysorbate 20, 60 and 80 (e.g., sold under the names Alkest^{®} and Tween 20, 60 and 80, made commercially available by suppliers like Oxiteno and Croda International, respectively) as well as PEG-6 caprylic/capric glyceride (e.g., sold under the names Softigen^{®} and Glycerox^{™}, made commercially available by IOI Oleo and Croda International, respectively). Other often desired nonionic surfactants include alkyl polyglucosides like cocoyl glucoside, decyl glucoside, hydroxystearyl glucoside, cetearyl glucoside, lauryl glucoside cetearyl isononanoate, cetearth-20, cetearyl alcohol, glyceryl stearate, ceteareth-12 or a mixture thereof.

Optional cationic surfactants suitable for use in the sanitizing composition for use according to the present invention include heterocyclic ammonium salts such as cetyl or stearyl pyridinium chloride, alkyl amidoethyl pyrrylinodium methyl sulfate, and lapyrium chlorideStill other types of optional cationic surfactants that may be used are the various ethoxylated quaternary amines and ester quats. Examples include PEG-5 stearyl ammonium lactate (e.g., Genamin KSL manufactured by Clariant), PEG-2 coco ammonium chloride, PEG-15 hydrogenated tallow ammonium chloride, PEG 15 stearyl ammonium chloride, dipalmitoyl ethyl methyl ammonium chloride, dipalmitoyl hydroxyethyl methyl sulfate, and stearyl amidopropyl dimethylamine lactate. Even other useful optional cationic surfactants suitable for use include quaternized hydrolysates of silk, wheat, and keratin proteins, and it is within the scope of the invention to use mixtures of the aforementioned optional cationic surfactants.

If used, optional surfactants collectively and typically will make up no more than 10.0% (often preferably no more than 8%) by weight of the sanitizing composition for use according to the present invention. When present, optional surfactants will preferably make no more than 5% by weight of the sanitizing composition for use according to the present invention. Often they make up from 0.001 to 4.5%, and more typically, from 0.01 to 4.0% by weight of the sanitizing composition for use according to the present invention, including all ranges subsumed therein. In an embodiment of the invention, optional surfactant makes up from 0.1 to 2% by weight of the sanitizing composition for use according to the present invention.

In another embodiment of the invention, optional surfactant used is a nonionic surfactant making up from 0.0 to 4%, and preferably, from 0.1 to 2%, and most preferably, from 0.3 to 1.5% by weight of the composition and including all ranges subsumed therein. In even another embodiment of the invention, only nonionic surfactant is used as the optional surfactant. Preferably, the nonionic surfactant used is a polysorbate, PEG-6 caprylic/capric glyceride, an alkyl polyglucoside or a mixture thereof.

In still another embodiment, lauramine oxide is a surfactant used in the composition for use according to the present invention at from 0.1 to 10% and preferably, from 0.2 to 8%, and most preferably, from 0.5 to 5% by weight of the composition.

The sanitizing composition for use according to the present invention comprises less than 0.5%, and preferably, less than 0.1% by weight anionic surfactant and most preferably, no (0.0% by weight) anionic surfactant like sodium lauryl sulfate, sodium laureth sulfate and/or sodium pareth sulfate. Thus, the sanitizing composition for use according to the present invention is most preferably sulfate free.

The pH of the sanitizing composition for use according to the present invention is typically from 3.0 to 8.2, and preferably, from 3.1 to 7.5, and most preferably, from 3.2 to 7.0. In an embodiment of the invention, the pH of the sanitizing composition for use according to the present invention is from 3.3 to 6.5, and preferably, from 3.3 to 5.5. Adjusters suitable to modify the pH of the sanitizing composition for use according to this invention may be used. Such pH adjusters include triethylamine, NaOH, KOH, H₂SO4, HCl, C₆H₈O₇ (i.e., citric acid) or a mixture thereof. Buffers, like potassium oxide, to stabilize pH are also suitable for use. The pH of the composition is assessed by using conventional instrumentation such as a pH meter made commercially available from Thermo Scientific^{®}.

As to optional skin benefit agents, these can be water and/or oil soluble and are preferably water soluble. Humectants, like water soluble polyols, are generally desired. These include sorbitol, glycerol (or glycerine), mannitol, xylitol, maltitol or a mixture thereof. Other humectants include propylene glycol, dipropylene glycol, polypropylene glycol (e.g., PPG-9), polyethylene glycol, hydroxypropyl sorbitol, hexylene glycol, 1,3-butylene glycol, 1,2-octane diol, 1,2-hexane diol, isoprene glycol, 1,2,6-hexanetriol, ethoxylated glycerol, propoxylated glycerol and mixtures thereof. Most preferred is glycerin, propylene glycol or a mixture thereof. In an embodiment of the invention, the polyol used is at least 50% by weight glycerol, based on total weight of the polyol used in the sanitizing composition for use according to the invention. In another embodiment of the invention, the polyol used is a mixture of glycerol and hexylene glycol, at a weight ratio of 1:5 to 5:1 ratio, and preferably, from 4:1 to 1:4, and most preferably, from 3:1 to 1:3, including all ratios subsumed therein.

Water soluble moisturizing agents are suitable for optional use. These include dicaprylyl carbonate as well as substituted ureas like hydroxymethyl urea, hydroxyethyl urea, hydroxypropyl urea; bis(hydroxymethyl)urea; bis(hydroxyethyl)urea; bis(hydroxypropyl)urea; N,N'-dihydroxymethyl urea; N,N'-di-hydroxyethyl urea; N,N'-dihydroxypropyl urea; N,N,N'-tri-hydroxyethyl urea; tetra(hydroxymethyl)urea; tetra(hydroxyethyl) urea; tetra(hydroxypropyl urea; N-methyl, N'-hydroxyethyl urea; N-ethyl-N'-hydroxyethyl urea; N-hydroxypropyl-N'-hydroxyethyl urea and N,N'dimethyl-N-hydroxyethyl urea or a mixture thereof. Where the term hydroxypropyl appears, the meaning is generic for either 3-hydroxy-n-propyl, 2-hydroxy-n-propyl, 3-hydroxy-i-propyl or 2-hydroxy-i-propyl radicals. Most preferred for optional use is hydroxyethyl urea. The same is available as a 50% aqueous liquid from Nouryon under the trademark Hydrovance^{®}.

Further optional water-soluble skin benefit agents suitable to use in the sanitizing composition for use according to this invention include acids, like amino acids, such as arginine, valine or histidine. Vitamins may be used like vitamin B₂, picolinamide, niacinamide (vitamin B₃), panthenol (vitamin B₅), vitamin B₆, vitamin C, mixtures thereof or the like. Derivatives, and especially, water soluble derivatives of such vitamins may also be employed. For instance, vitamin C derivatives such as ascorbyl tetraisopalmitate, magnesium ascorbyl phosphate and ascorbyl glycoside may be used alone or in combination with each other. Other skin benefit agents suitable for use include hyaluronic acid and salts thereof (like Na+ and K+ salts of the same) 4-ethyl resorcinol, extracts like sage, aloe vera, green tea, sugar cane, citrus, grapeseed, thyme, chamomile, yarrow, cucumber, liquorice, rosemary extract or a mixture thereof. Water soluble sunscreens like ensulizole may also be used as may electrolytes such as NaCl and/or KCI. Total amount of optional water-soluble benefit agents (including mixtures) when present in the composition for use according to the invention may range from 0.0001 to 10%, preferably. from 0.001 to 6.5%, and most preferably, from 0.01 to 3.5% by weight, based on total weight of the sanitizing composition and including all ranges subsumed therein. In a preferred embodiment of the invention, glycerol may be used in the sanitizing composition for use according to the invention at an amount from 0.05 to 2.0%, and a hydroxyethyl urea-based moisturizer, like Hydrovance, is optionally used at an amount from 0.05 to 1.5%, and hexylene glycol is optionally used at an amount from 1 to 4% by weight, based on total weight of the composition and including all ranges subsumed therein.

In an often desired embodiment, the sanitizing composition for use according to the present invention does comprise from 0.5 to 10%, and preferably, from 0.6 to 7%, and most preferably, from 0.8 to 3.5% by weight niacinamide (for longer lasting antimicrobial benefit) based on total weight of the sanitizing composition. In still another embodiment, niacinamide is present from 0.7 to 1.4% by weight of the composition.

It is also within the scope of the invention to optionally include oil soluble benefit agents. Illustrative examples of the types of oil soluble benefit agents that may optionally be used in the sanitizing composition for use according to this invention include components like stearic acid, vitamins like vitamin A, D, E and K (and their oil soluble derivatives), sunscreens like ethylhexylmethoxycinnamate, bis-ethyl hexyloxyphenol methoxyphenol triazine, 2-ethylhexyl-2-cyano-3,3-diphenyl-2-propanoic acid, drometrizole trisiloxane, 3,3,5-trimethyl cyclohexyl 2-hydroxybenzoate, 2-ethylhexyl-2-hydroxybenzoate or a mixture thereof.

Other optional oil soluble benefit agents suitable for use include resorcinols like 4-hexyl resorcinol, 4-phenylethyl resorcinol, 4-cyclopentyl resorcinol, 4-cyclohexyl resorcinol 4-isopropyl resorcinol or a mixture thereof. Also, 5-substituted resorcinols like 4-cyclohexyl-5-methylbenzene-1,3-diol, 4-isopropyl-5-methylbenzene-1,3-diol, mixtures thereof or the like may be used. The 5-substituted resorcinols, and their synthesis are described in commonly assigned U.S. Published Patent Application No. 2016/0000669A1.

Even other oil soluble benefit agents suitable for use include omega-3 fatty acids, omega-6 fatty acids, climbazole, magnolol, honokiol, farnesol, ursolic acid, myristic acid, geranyl geraniol, oleyl betaine, cocoyl hydroxyethyl imidazoline, hexanoyl sphingosine, 12-hydroxystearic acid, petroselinic acid, conjugated linoleic acid, stearic acid, palmitic acid, lauric acid, terpineol, thymol essential components, the dissolution auxiliary selected from limonene, pinene, camphene, cymene, citronellol, citronellal, geraniol, nerol, linalool, rhodinol, borneol, isoborneol, menthone, camphor, safrole, isosafrole, eugenol, isoeugenol, tea tree oil, eucalyptus oil, peppermint oil, neem oil, lemon grass oil, orange oil, bergamot oil, mixtures thereof or the like.

Another optional oil soluble benefit agent that may be used is a retinoic acid precursor. In one embodiment of the invention, the retinoic acid precursor is retinol, retinal, retinyl propionate, retinyl palmitate, retinyl acetate or a mixture thereof. Retinyl propionate, retinyl palmitate and mixtures thereof are typically preferred. Still another retinoic acid precursor suitable for use is hydroxyanasatil retinoate made commercially available under the name Retextra^{®} as supplied by Molecular Design International. The same may be used in a mixture with the oil soluble benefit agents described herein.

When optional (i.e., 0.0 to 1.5% by weight) oil soluble benefit agent is used in the sanitizing composition for use according to the invention, it typically makes up from 0.001 to 1.3%, and in another embodiment, from 0.05 to 1.2%, and in yet another embodiment, from 0.1 to 0.5% by weight of the total weight of the composition, including all ranges subsumed therein.

Film forming agents may be used in the compositions for use according to the present invention. While optional, such agents can aid with composition adhering to the surface they are applied to. Film forming agents include those having hydrophilic properties and they include materials comprising polyvinylpyrrolidone (PVP), acrylates, acrylamides, and copolymers thereof. Deposition agents like organosiloxanes may also be used. When used, such agents make up from 0.001 to 1% by weight of the sanitizing composition for use according to the invention.

Other optional components suitable for use in the composition for use according to the present invention are anti-mosquito agents like eucalyptus oil, lavender oil, N,N-diethyl-meta-toluamide (DEET), a mixture thereof or the like. Even other ingredients which may be used include octopirox (piroctone), zinc pyrithione, chloroxylenol, triclosan, cetylpyridinium chloride as well as silver compounds including silver oxide, nitrate, sulfate, phosphate, carbonate, acetate, benzoate, a mixture thereof or the like. If used, these other components typically make up from 0.001 to 1.6%, and preferably, from 0.01 to 1.2% by weight of the sanitizing composition for use according to the present invention.

Preservatives may be used in the sanitizing composition for use according tof the present invention. When the sanitizing composition is prepared by preparing a precursor and diluting the precursor with water, for example on demand, preservatives could be included but they are not required since the resulting sanitizing composition for use according to the present invention would be used shortly after preparation. If, however, precursor is diluted with water and the resulting sanitizing composition is filled into a refill package (such as a sustainable refill spray bottle) preservative is typically recommended. Precursor will typically have less than 15% by weight water, and preferably, less than 10% by weight water, and most preferably, from 1 to 7% by weight water, based on total weight of the sanitizing precursor and including all ranges subsumed therein.

When the sanitizing precursor is diluted with water and mixed or agitated, the resulting sanitizing composition for use according to the present invention comprising at least 80% by weight water is recovered or prepared and is capable of delivering a bacteria log kill of at least 2 and a viral log inactivation of at least 2 in less than 3 minutes after topical application.

As to illustrative preservatives suitable for use, these include sodium benzoate, iodopropynyl butyl carbamate, phenoxyethanol, hydroxyacetophenone, ethylhexylglycerine, methyl paraben, propyl paraben, imidazolidinyl urea, sodium dehydroacetate, dimethyl-dimethyl (DMDM) hydantoin and benzyl alcohol or a mixture thereof. Other preservatives suitable for use include sodium dehydroacetate, chlorophenesin and decylene glycol. Preservatives are preferably employed in amounts ranging from 0.01% to 2.0% by weight of the total weight of sanitizing composition for use according to the present invention, including all ranges subsumed therein. Also preferred is a preservative system with hydroxyacetophenone alone or in a mixture with other preservatives.

Fragrances, fixatives, opacifiers (like titanium dioxide), chelators (like EDTA) may optionally be included in the sanitizing composition for use according to the present invention. Each of these substances may range from about 0.03 to 3%, preferably, between 0.1 and 2.6% by weight of the total weight of the sanitizing composition, including all ranges subsumed therein.

Another optional additive suitable for use includes hemp oil with 2.5 to 25% by weight cannabigerol and/or cannabidiol at from 0.5 to 10 percent by weight. When used, such oil makes up from 0.0001 to 1.5% by weight of the sanitizing composition for use according to the invention, and preferably, from 0.01 to 1% by weight of the sanitizing composition for use according to the present invention, , including all ranges subsumed therein.

Unexpectedly, the sanitizing composition for use according to the present invention results in at least a log kill of at least 2 in under 3 minutes, and preferably, at least a log kill of 3 in under 2 minutes, and most preferably at least a log kill of 4.7 in under 1.2 minutes after application (i.e., contact) with a bacteria colony having at least one of *Staphylococus aureus, Escherichia coli and Cutibacterium acnes.* Additionally, a viral log inactivation of at least 2 in less than 3 minutes was observed after topical application to a surface with *adenovirus,* and preferably, at least 2.4 in less than 1.5 minutes after topical application to *adenovirus.*

The sanitizing composition for use according to this invention can be provided as a micellar water (surfactant forming micelles in water, preferably cationic surfactant) that surprisingly provides excellent antimicrobial and viral activity reduction upon contact with a surface. When making the sanitizing composition for use according tof the invention, ingredients are mixed with moderate shear, under atmospheric conditions and at a temperature from 20 to 65°C. If preparing from a precursor, depending on the desired volume, precursor (with less than 15% by weight water) should be mixed with water followed by moderate agitation to produce desired sanitizing composition for use according to the invention.

Typically, the viscosity of the sanitizing composition for use according to the invention will be under 30 Pa.s (30,000 cps). Often the viscosity of the sanitizing composition will be from 0 to 25 (25,000), and preferably, from 0 to 18 (18,000), and most preferably, from 0 to 14 Pa.s (14,000 cps), including all ranges subsumed therein. In an embodiment of the invention, the viscosity of the sanitizing composition for use according to the invention is from 0.001 to 3 Pa.s (1 to 3,000 cps), and in still another embodiment of the invention, the viscosity of the sanitizing composition for use according to the invention is from 0.001 to 2 Pa.s (1 to 2,000 cps). In yet another embodiment of the invention, the viscosity of the sanitizing composition is from 0.001 to 0.25 Pa.s (1 to 250 cps).

To adjust sanitizing composition viscosity, it is within the scope of the invention to optionally include thickening agents. Optionally suitable for use in sanitizing composition for use according to the present invention are thickeners classified as polysaccharides. Examples include fibers, starches, natural/synthetic gums and cellulosics. Representative of the starches are chemically modified starches such as sodium hydroxypropyl starch phosphate and aluminum starch octenylsuccinate. Tapioca starch is often preferred, as is maltodextrin. Suitable gums include xanthan, tara, sclerotium, pectin, karaya, arabic, agar, guar (including Acacia senegal guar), carrageenan, alginate and combinations thereof. Suitable cellulosics include hydroxypropyl cellulose, hydroxypropyl methylcellulose, ethylcellulose, sodium carboxy methylcellulose (cellulose gum/carboxymethyl cellulose) and cellulose (e.g. cellulose microfibrils, cellulose nanocrystals or microcrystalline cellulose). Sources of cellulose microfibrils include secondary cell wall materials (e.g. wood pulp, cotton), bacterial cellulose, and primary cell wall materials. Preferably the source of primary cell wall material is selected from parenchymal tissue from fruits, roots, bulbs, tubers, seeds, leaves and combination thereof; more preferably is selected from citrus fruit, tomato fruit, peach fruit, pumpkin fruit, kiwi fruit, apple fruit, mango fruit, sugar beet, beet root, turnip, parsnip, maize, oat, wheat, peas and combinations thereof; and even more preferably is selected from citrus fruit, tomato fruit and combinations thereof. A most preferred source of primary cell wall material is parenchymal tissue from citrus fruit. Citrus fibers, such as those made available by Herbacel^{®} as AQ Plus can also be used as source for cellulose microfibrils. The cellulose sources can be surface modified by any of the known methods including those described in Colloidal Polymer Science, Kalia et al., "Nanofibrillated cellulose: surface modification and potential applications" (2014), Vol 292, Pages 5-31.

Synthetic polymers are yet another class of effective thickening agent. This category includes crosslinked polyacrylates such as the Carbomers, acrylate copolymers, acrylates/ acrylate (C₁₀-C₃₀) alkyl acrylate crosspolymers, polyacrylamides such as Sepigel^{®} 305 and taurate copolymers such as Simulgel^{®} EG and Aristoflex^{®} AVC, the copolymers being identified by respective INCI nomenclature as Sodium Acrylate/Sodium Acryloyldimethyl Taurate and Acryloyl Dimethyltaurate/Vinyl Pyrrolidone Copolymer. Another preferred synthetic polymer suitable for thickening is an acrylate-based polymer made commercially available by Seppic and sold under the name Simulgel INS100. Calcium carbonate, fumed silica, and magnesium-aluminum-silicate may also be used.

The amounts of the thickening agent, when used, may range from 0.001 to 5%, by weight of the sanitizing composition for use according to the invention. Maltodextrin, xanthan gum, and carboxymethyl cellulose are the often preferred thickening agents. Other thickening agents often desired are dilinoleyl/dimethyl carbonate copolymer, stearyl alkonium hectorite, cetyl (cetearyl, stearyl) fatty alcohols, tara gum, pentaerythrityl tetrastearate or a mixture thereof.

As to packaging, the sanitizing composition for use according to the present invention, can be packaged in a spray bottle, bottle with a self-foaming mechanism/pump, or provided as an impregnating wetting agent on cotton swabs, wipes, towelettes, cosmetic substrate sheets (like those described in US 6,294,182 B1) or the like. As the viscosity is increased with thickening agent, the sanitizing composition for use according to the invention may be provided to consumers in a squeeze bottle, bottle with pump, or tube as a gel composition. The spray bottle may also be metal and the sanitizing composition may be provided via conventional aerosol packaging technologies and including those which utilize air-in-bag discharging cannisters, mechanisms and actuators. It is also within the scope of the invention to include foaming agents (e.g., zwitterionic and/or amphoteric surfactants) so that the sanitizing composition for use according to the invention can be discharged as a foam. In an embodiment of the invention, the sanitizing composition for use according to the invention is provided in a standard spray bottle with a spray actuator and sprayed on to the surface needing sanitizing, especially skin. The sanitizing composition for use according to the invention is particularly useful in preventing adverse skin reactions (like acne development) caused by microbes that collect under personal protective equipment (including pants, gowns, and especially, protective face masks). Thus, it is advisable to apply the sanitizing composition for use according to the present invention to skin prior to putting on personal protective equipment (and even throughout the day when personal protective equipment is being worn).

The sanitizing composition for use according to the invention should be supplied with instructions to apply (preferably spray) the composition on to a surface, like skin, for bacteria kill and viral activity reduction. Precursor will preferably be provided for in biodegradable packaging and the packaging used is preferably refillable or reusable, biodegradable, and/or at least 50%, and preferably, at least 100% made from post-consumer recycled resin.

The following Examples are provided to further illustrate an understanding of the invention. The Examples are not intended to limit the scope of the claims.

### Examples

The ingredients set out in the tables were mixed using moderate shear under atmospheric conditions with temperature set at 30°C. The resulting formulae were recovered and assessed. Testing was conducted by experts familiar with the identified ASTM methods as summarized below.

### ASTM E2783-11(Reapproved 2016): Test Method Used to Assess Antimicrobial Activity of Miscible Compounds

A dilution/aliquot of the test material was brought into contact with a known population of test organisms (*Staphylococcus aureus and Escherichia coli*) for the specified periods of time as defined below and where temperature was 25°C . The activity of the test material was quenched at the specified sampling intervals with an appropriate neutralizing technique using a solution of Tween 80 (10%) and lecithin (1%).

The test material/organisms were neutralized at the times identified and the surviving microorganisms enumerated. The percent and log₁₀ reduction, from an initial microbial population was calculated.

### ASTM E1052-20: Test method Used to Assess Microbicides against Viruses in Suspension

Virus suspension was added to nine parts of test substance, suspension charged with *Adenovirus.* The resulting mixture was held at a temperature of 25°C for the identified contact times and then assayed for viable virus in an a host system. For a control, one part of virus is added to nine parts of buffer harmless to the virus and any host cells. Cell culture, cytotoxicity, and virus susceptibility controls were included in each test.

### Example 1

The below sanitizing composition was made for use according to the invention. The trimonium compound used was cetrimonium chloride. The composition had a viscosity of about 0.1 Pa.s (100 cps) and was applied to the hands of panelists via a spray bottle. The composition was mild and non-irritating after application.

| **Ingredients** | **Weight (%) active** |
|---|---|
| Water | balance |
| Natural Fragrance Extract | 0.01 |
| Sodium Chloride | 0.01 |
| D--Panthenol (75%) | 0.01 |
| Niacinamide | 0.01 |
| Sodium Ascorbyl Phosphate | 0.01 |
| Citric Acid | 0.025 |
| Humectant | 1 |
| Cetrimonium Chloride | 0.1 |
| PEG-6-Caprylic/Capric Glyceride | 0.5 |
| Cetylpyridinium Chloride | 0.02 |
| Glycol | 2.5 |
| EDTA | 0.05 |
| Potassium Chloride | 0.01 |
| Preservative | 0.2 |

### Example 2

The sanitizing composition below was made for use according to the invention. The trimonium compound used was cetrimonium chloride. The composition had a viscosity of about 0.1 Pa.s (100 cps) and was impregnated into polyester wipes/towelettes. The composition was mild and non-irritating after application to panelists hands and face via the wipes/towelettes.

| **Ingredients** | **Weight (%)** |
|---|---|
| Water | balance |
| Sodium Citrate Dihydrate | 0.5 |
| 1,2-propanediol | 1 |
| Sodium benzoate | 0.5 |
| Glycerin | 0.3 |
| Moisturizer | 0.1 |
| Vitamin E Acetate | 0.01 |
| Cetrimonium Chloride | 0.145 |
| Nonionic emulsifier | 3.2 |
| Citric Acid | 0.25 |

### Example 3

Sanitizing Composition for use according to the invention with Fragrance

| Ingredients | Weight % |
|---|---|
| Water | Balance |
| Tetrasodium EDTA | 0.05 |
| Sodium Chloride | 0.01 |
| Potassium Chloride | 0.01 |
| Panthenol | 0.01 |
| NIACINAMIDE | 0.01 |
| Citric Acid | 0.025 |
| Hexylene Glycol | 2.5 |
| Hydroxyethyl Urea | 0.08 |
| pH adjuster | 0.001 |
| moisturizer | 0.004 |
| pH stabilizer | 0.006 |
| Glycerin | 1.0 |
| PEG-6-Caprylic/Capric Glycerides | 0.5 |
| Cetrimonium Chloride | 0.1 |
| Phenoxyethanol | 0.2 |
| Cetylpyridinium Chloride | 0.02 |
| Propylene Glycol | 0.03 |
| Sunscreen | 0.006 |
| Trideceth-9 | 0.07 |
| PEG-40 Hydrogenated Castor Oil | 0.07 |
| Fragrance | 0.05 |

### Example 4

Sanitizing Composition for use according to the Invention, Fragrance Free

| Ingredient INCI Name | Ingredient % in formula |
|---|---|
| Water | Balance |
| Tetrasodium EDTA | 0.051 |
| Sodium Chloride | 0.01 |
| Potassium Chloride | 0.01 |
| Panthenol | 0.01 |
| Niacinamide | 0.01 |
| Citric Acid | 0.025 |
| Hexylene Glycol | 2.5 |
| Hydroxyethyl Urea | 0.08 |
| Ethanolamine | 0.001 |
| Moisturizer | 0.006 |
| Ammonium Lactate | 0.004 |
| Glycerin | 1.0 |
| PEG-6-Caprylic/Capric Glycerides | 0.5 |
| Cetrimonium Chloride | 0.1 |
| Phenoxyethanol | 0.2000 |
| Cetylpyridinium Chloride | 0.02 |
| Glycol | 0.03 |

### Example 5

### Composition Assessment

**Fragrance Free Sanitizing Composition-Table A**

| Composition of Example 4 | Replicate | Test organism: S.aureus ATCC6538 Log₁₀ Reduction-Contact Time (n=3) | | | | | | | |
|---|---|---|---|---|---|---|---|---|---|
| | | 30 Seconds | | | | 60 Seconds | | | |
| | | Reduction | | Average | | Reduction | | Average | |
| | | Log₁₀ | Percent(%) | Log₁₀ | Percent(%) | Log₁₀ | Percent(%) | Log₁₀ | Percent(%) |
| | 1 | 3.84 | 99.99 | 3.94 | 99.99 | 4.90 | 99.99 | 4.93 | 99.99 |
| | 2 | 4.08 | 99.99 | | | 5.04 | 99.99 | | |
| | 3 | 3.89 | 99.99 | | | 4.86 | 99.99 | | |

The data in Table A unexpectedly shows a Log₁₀ average reduction of 3.94, 99.99 percent S.aureus reduction after 30 seconds of contact and a Log₁₀ average reduction of 4.93, 99.99 percent S.aureus reduction after 60 seconds of contact, even with trimonium compound at 0.1% and over 80% by weight water in the composition.

**Fragrance Comprising Sanitizing Composition-Table B**

| Composition of Example 3 | Replicate | Test organism: E.coli ATCC25922 Log₁₀ Reduction-Contact Time (n=3) | | | | | | | |
|---|---|---|---|---|---|---|---|---|---|
| | | 30 Seconds | | | | 60 Seconds | | | |
| | | Reduction | | Average | | Reduction | | Average | |
| | | Log₁₀ | Percent(%) | Log₁₀ | Percent(%) | Log₁₀ | Percent(%) | Log₁₀ | Percent(%) |
| | 1 | 5.83 | 99.99 | 5.53 | 99.99 | >5.83 | 99.99 | 5.83 | 99.99 |
| | 2 | 5.53 | 99.99 | | | >5.83 | 99.99 | | |
| | 3 | 5.23 | 99.99 | | | 5.83 | 99.99 | | |

The data in Table B unexpectedly shows a Log₁₀ average reduction of 5.53, 99.99 percent E.coli reduction after 30 seconds of contact and a Log₁₀ average reduction of 5.83, 99.99 percent E.coli reduction after 60 seconds of contact, even with trimonium compound at 0.1% and over 80% by weight water in the composition.

**Fragrance Comprising Sanitizing Composition-Table C**

| Composition of Example 3 | Replicate | Test organism: S.aureus ATCC6538 Log₁₀ Reduction-Contact Time (n=3) | | | | | | | |
|---|---|---|---|---|---|---|---|---|---|
| | | 30 Seconds | | | | 60 Seconds | | | |
| | | Reduction | | Average | | Reduction | | Average | |
| | | Log₁₀ | Percent(%) | Log₁₀ | Percent(%) | Log₁₀ | Percent(%) | Log₁₀ | Percent(%) |
| | 1 | 3.83 | 99.99 | 3.91 | 99.99 | 4.84 | 99.99 | 4.82 | 99.99 |
| | 2 | 3.91 | 99.99 | | | 4.91 | 99.99 | | |
| | 3 | 3.99 | 99.99 | | | 4.70 | 99.99 | | |

The data in Table C unexpectedly shows a Log₁₀ average reduction of 3.91, 99.99 percent S.aureus reduction after 30 seconds of contact and a Log₁₀ average reduction of 4.82, 99.99 percent S.aureus reduction after 60 seconds of contact, even with trimonium compound at 0.1% and over 80% by weight water in the composition.

**Fragrance Free Sanitizing Composition-Table D**

| Composition of Example 4 | Replicate | Test organism: E.coli ATCC25922 Log₁₀ Reduction-Contact Time (n=3) | | | | | | | |
|---|---|---|---|---|---|---|---|---|---|
| | | 30 Seconds | | | | 60 Seconds | | | |
| | | Reduction | | Average | | Reduction | | Average | |
| | | Log₁₀ | Percent(%) | Log₁₀ | Percent(%) | Log₁₀ | Percent(%) | Log₁₀ | Percent(%) |
| | 1 | 5.83 | 99.99 | 5.64 | 99.99 | >5.83 | 99.99 | >5.83 | 99.99 |
| | 2 | 5.53 | 99.99 | | | >5.83 | 99.99 | | |
| | 3 | 5.55 | 99.99 | | | >5.83 | 99.99 | | |

The data in Table D unexpectedly shows a Log₁₀ average reduction of 5.64, 99.99 percent E.coli reduction after 30 seconds of contact and a Log₁₀ average reduction of 5.83, 99.99 percent E.coli reduction after 60 seconds of contact, even with trimonium compound at 0.1% and over 80% by weight water in the composition.

**Table E**

| **ADENOVIRUS Inactivation-60 Seconds** | | | | | |
|---|---|---|---|---|---|
| | Control | CTAB (0.05%) | CTAB (0.1%) | CPC (0.05%) | CPC (0.1%) |
| Log₁₀ TCID 50/0.1 ml | 4.75 | 2.5 | 1.75 | 1.6 | 1.6 |

The data in Table E unexpectedly shows that trimonium compound (e.g., cetrimonium bromide (CTAB)) surprisingly had an inactivation of Adenovirus via the Log₁₀ TCID method comparable to what was observed with cetylpyridinum chloride (CPC) at 60 seconds.

### Example 6

A composition similar to the one made in Example 3 was prepared except that 0.05% by weight of both benzalkonium chloride and didecyldimonium chloride were used in lieu of cetrimonium chloride.

The data obtained using the aforementioned ASTM tests unexpectedly showed a Log₁₀ reduction of about 4.6, 99.99 percent E.coli reduction after 10 seconds of contact and a Log₁₀ reduction of greater than 5, 99.99 percent E.coli reduction after 60 seconds of contact.

The data also revealed a Log₁₀ reduction of about 4, 99.99 percent S.aureus after 10 seconds of contact and a Log₁₀ reduction of about 4.9, 99.99 percent S.aureus after 60 seconds of contact.

### Example 7

A composition similar to the one made in Example 3 was prepared except that 0.15% by weight benzalkonium chloride was used in lieu of cetrimonium chloride.

Surprisingly the composition had a Log₁₀ TCID viral inactivation reduction of greater than 2 in under 3 minutes.

## Claims

1. Non-therapeutic use, as an antibacterial and antiviral sanitizing composition, of a composition comprising:
a) at least 80% by weight water; and
b) a cationic surfactant
wherein the composition is capable of delivering a bacteria log kill of at least 2 and a viral log inactivation of at least 2 in less than 3 minutes after topical application and further wherein the composition has less than 10% by weight of ethanol and when total surfactant in the composition is more than 4.5% by weight and cationic surfactant in the composition includes a combination of both cetrimonium chloride and benzalkonium chloride, benzalkonium chloride makes up at least 16.5% by weight of the combination; wherein the composition has less than 0.3% by weight benzalkonium chloride and/or benzethonium chloride; wherein the cationic surfactant is 95 to 100% by weight trimonium compound, dimonium compound or both; wherein the composition comprises 0.0% by weight aliphatic alcohol excluding ethanol and not including diols and polyols; wherein the trimonium compound is an alkyl trimonium compound comprising cetrimonium chloride, cetrimonium bromide, mytrimonium chloride, mytrimonium bromide, behentrimonium methosulfate, cocotrimonium methosulfate, behentrimonium chloride, behentrimonium bromide, steartrimonium chloride, steartrimonium bromide, laurtrimonium chloride, laurtrimonium bromide or a mixture thereof, and wherein the dimonium compound is distearyl dimonium chloride, didecyl dimonium chloride, dicoco dimonium chloride, a mixture thereof or benzethonium chloride and/or benzalkonium chloride, and wherein cationic surfactant makes up from 0.08 to 0.4% by weight of the composition.

2. Use according to claim 1 wherein the cationic surfactant comprises cetrimonium chloride, cetrimonium bromide or a mixture thereof.

3. Use according to claim 1 or 2 wherein the cationic surfactant is cetrimonium chloride.

4. Use according to any of claims 1 to 3 wherein the composition further comprises a nonionic surfactant.

5. Use according to any of claims 1 to 4 wherein the composition further comprises a polyol and the polyol is sorbitol, glycerol, mannitol, xylitol, maltitol dipropylene glycol, polypropylene glycol, polyethylene glycol, hydroxypropyl sorbitol, hexylene glycol, 1,3-butylene glycol, 1,2-octane diol, 1,2-hexane diol, isoprene glycol, 1,2,6-hexanetriol, ethoxylated glycerol, propoxylated glycerol or a mixture thereof.

6. Use according to any of claims 1 to 5 wherein the composition further comprises soluble omega-3 fatty acids, omega-6 fatty acids, climbazole, magnolol, honokiol, farnesol, ursolic acid, myristic acid, geranyl geraniol, oleyl betaine, cocoyl hydroxyethyl imidazoline, hexanoyl sphingosine, 12-hydroxystearic acid, petroselinic acid, conjugated linoleic acid, stearic acid, palmitic acid, lauric acid, terpineol, thymol essential components, the dissolution auxiliary selected from limonene, pinene, camphene, cymene, citronellol, citronellal, geraniol, nerol, linalool, rhodinol, borneol, isoborneol, menthone, camphor, safrole, isosafrole, eugenol, isoeugenol, tea tree oil, eucalyptus oil, peppermint oil, neem oil, lemon grass oil, orange oil, bergamot oil or a mixture thereof.

7. Use according to any claims 1 to 6 wherein the composition further comprises arginine, valine, histidine, vitamin B₂, niacinamide (vitamin B₃), vitamin B₅, vitamin B₆, vitamin C, ascorbyl tetraisopalmitate, magnesium ascorbyl phosphate, ascorbyl glycoside, hyaluronic acid and salts thereof, 4-ethyl resorcinol, sage, aloe vera, green tea, sugar cane, citrus, grapeseed, thyme, chamomile, yarrow, cucumber, liquorice, rosemary, ensulizole, NaCl, KCI or a mixture thereof.

8. Use according to any of claims 1 to 7 wherein the composition further comprises hydroxymethyl urea, hydroxyethyl urea, hydroxypropyl urea; bis(hydroxymethyl)urea; bis(hydroxyethyl)urea; bis(hydroxypropyl)urea; N,N'-dihydroxymethyl urea; N,N'-di-hydroxyethyl urea; N,N'-di-hydroxypropyl urea; N,N,N'-tri-hydroxyethyl urea; tetra(hydroxymethyl) urea; tetra(hydroxyethyl) urea; tetra(hydroxypropyl urea; N-methyl, N'-hydroxyethyl urea; N-ethyl-N'-hydroxyethyl urea; N-hydroxypropyl-N'-hydroxyethyl urea and N,N'dimethyl-N-hydroxyethyl urea or a mixture thereof.

9. Use according to any of claims 1 to 8 wherein the composition further comprises eucalyptus oil, lavender oil, N,N-diethyl-meta-toluamide (DEET), octopirox (piroctone), zinc pyrithione, benzethonium chloride, benzalkonium chloride, chloroxylenol, triclosan, cetylpyridinium chloride, silver oxide, silver nitrate, silver sulfate, silver phosphate, silver carbonate, silver acetate, silver benzoate or a mixture thereof.

10. Use according to any of claims 1 to 9 wherein the composition comprises at least 82% water.

11. Use according to any of claims 1 to 10 wherein the composition comprises from 85 to 99.5%by weight water and less than 5% by weight ethanol.

12. Use according to any of claims 1 to 11 wherein the composition further comprises glycerol and has a viscosity under 30 Pa.s (30,000 cps).

13. Use according to any of claims 1 to 12 wherein the composition further comprises an anti-mosquito agent, octopirox, zinc pyrithione, chloroxylenol, triclosan, cetylpyridinium chloride or a silver compound which is a silver oxide, nitrate, sulfate, phosphate, carbonate, acetate, benzoate or a mixture thereof.

14. A composition comprising:
a) at least 80% by weight water; and
b) a cationic surfactant
wherein the composition is capable of delivering a bacteria log kill of at least 2 and a viral log inactivation of at least 2 in less than 3 minutes after topical application and further wherein the composition has less than 10% by weight) of ethanol and when total surfactant in the composition is more than 4.5% by weight and cationic surfactant in the composition includes a combination of both cetrimonium chloride and benzalkonium chloride, benzalkonium chloride makes up at least 16.5% by weight of the combination; wherein the composition has less than 0.3% by weight benzalkonium chloride and/or benzethonium chloride; wherein the cationic surfactant is 95 to 100% by weight trimonium compound, dimonium compound or both; wherein the composition comprises 0.0% by weight aliphatic alcohol excluding ethanol and not including diols and polyols; wherein the trimonium compound is an alkyl trimonium compound comprising cetrimonium chloride, cetrimonium bromide, mytrimonium chloride, mytrimonium bromide, behentrimonium methosulfate, cocotrimonium methosulfate, behentrimonium chloride, behentrimonium bromide, steartrimonium chloride, steartrimonium bromide, laurtrimonium chloride, laurtrimonium bromide or a mixture thereof, and wherein the dimonium compound is distearyl dimonium chloride, didecyl dimonium chloride, dicoco dimonium chloride, a mixture thereof or benzethonium chloride and/or benzalkonium chloride, and wherein cationic surfactant makes up from 0.08 to 0.4% by weight of the composition, for use in a method of preventing adverse skin reactions like acne development caused by microbes that collect under personal protective equipment comprising the step of applying the composition to skin prior to putting on personal protective equipment.

## Patentansprüche

1. Nicht-therapeutische Verwendung einer Folgendes umfassenden Zusammensetzung als antibakterielle und antivirale Desinfektionszusammensetzung:
a) mindestens 80 Gew.-% Wasser; und
b) ein kationisches Tensid,
wobei die Zusammensetzung in der Lage ist, innerhalb von weniger als 3 Minuten nach topischer Anwendung eine bakterielle Log-Abtötung von mindestens 2 und eine virale Log-Inaktivierung von mindestens 2 zu erzielen, und wobei die Zusammensetzung ferner weniger als 10 Gew.-% Ethanol aufweist und wenn die Gesamtmenge an Tensid in der Zusammensetzung mehr als 4,5 Gew.-% beträgt und das kationische Tensid in der Zusammensetzung eine Kombination aus sowohl Cetrimoniumchlorid als auch Benzalkoniumchlorid umfasst, Benzalkoniumchlorid mindestens 16,5 Gew.-% der Kombination ausmacht; wobei die Zusammensetzung weniger als 0,3 Gew.-% Benzalkoniumchlorid und/oder Benzethoniumchlorid aufweist; wobei das kationische Tensid 95 bis 100 Gew.-% Trimoniumverbindung, Dimoniumverbindung oder beides aufweist; wobei die Zusammensetzung 0,0 Gew.-% aliphatischen Alkohol umfasst, ausgenommen Ethanol und nicht umfassend Diole und Polyole; wobei die Trimoniumverbindung eine Alkyl-Trimoniumverbindung ist, umfassend Cetrimoniumchlorid, Cetrimoniumbromid, Mytrimoniumchlorid, Mytrimoniumbromid, Behentrimoniummethosulfat, Cocotrimoniummethosulfat, Behentrimoniumchlorid, Behentrimoniumbromid, Steartrimoniumchlorid, Steartrimoniumbromid, Laurtrimoniumchlorid, Laurtrimoniumbromid oder eine Mischung davon, und wobei die Dimoniumverbindung Distearyldimoniumchlorid, Didecyldimoniumchlorid, Dicocodimoniumchlorid, eine Mischung davon oder Benzethoniumchlorid und/oder Benzalkoniumchlorid ist, und wobei das kationische Tensid 0,08 bis 0,4 Gew.-% der Zusammensetzung ausmacht.

2. Verwendung nach Anspruch 1, wobei das kationische Tensid Cetrimoniumchlorid, Cetrimoniumbromid oder eine Mischung davon umfasst.

3. Verwendung nach Anspruch 1 oder 2, wobei das kationische Tensid Cetrimoniumchlorid ist.

4. Verwendung nach einem der Ansprüche 1 bis 3, wobei die Zusammensetzung zusätzlich ein nichtionisches Tensid enthält.

5. Verwendung nach einem der Ansprüche 1 bis 4, wobei die Zusammensetzung ferner ein Polyol umfasst und das Polyol Sorbit, Glycerin, Mannit, Xylit, Maltit, Dipropylenglykol, Polypropylenglykol, Polyethylenglykol, Hydroxypropylsorbit, Hexylenglykol, 1,3-Butylenglykol, 1,2-Octandiol, 1,2-Hexandiol, Isoprenglykol, 1,2,6-Hexantriol, ethoxyliertes Glycerin, propoxyliertes Glycerin oder eine Mischung davon.

6. Verwendung nach einem der Ansprüche 1 bis 5, wobei die Zusammensetzung ferner lösliche Omega-3-Fettsäuren, Omega-6-Fettsäuren, Climabazol, Magnolol, Honokiol, Farnesol, Ursolsäure, Myristinsäure, Geranylgeraniol, Oleylbetain, Cocoylhydroxyethylimidazolin, Hexanoylsphingosin, 12-Hydroxystearinsäure, Petroselinsäure, konjugierte Linolsäure, Stearinsäure, Palmitinsäure, Laurinsäure, Terpineol, wesentliche Bestandteile von Thymol, Auflösungshilfsmittel, ausgewählt unter Limonen, Pinen, Camphen, Cymol, Citronellol, Citronellal, Geraniol, Nerol, Linalool, Rhodinol, Borneol, Isoborneol, Menthon, Kampfer, Safrol, Isosafrol, Eugenol, Isoeugenol, Teebaumöl, Eukalyptusöl, Pfefferminzöl, Neemöl, Zitronengrasöl, Orangenöl, Bergamotteöl oder einer Mischung davon ausgewähltes Lösungshilfsmittel.

7. Verwendung nach einem der Ansprüche 1 bis 6, wobei die Zusammensetzung zusätzlich umfasst Arginin, Valin, Histidin, Vitamin B2, Niacinamid (Vitamin B3), Vitamin B5, Vitamin B6, Vitamin C, Ascorbyltetraisopalmitat, Magnesiumascorbylphosphat, Ascorbylglykosid, Hyaluronsäure und deren Salze, 4-Ethylresorin, Salbei, Aloe Vera, Grünen Tee, Zuckerrohr, Zitrus, Traubenkern, Thymian, Kamille, Schafgarbe, Gurke, Süßholz, Rosmarin, Ensulizol, NaCl, KCI oder eine Mischung davon.

8. Verwendung nach einem der Ansprüche 1 bis 7, wobei die Zusammensetzung ferner umfasst Hydroxymethylharnstoff, Hydroxyethylharnstoff, Hydroxypropylharnstoff; Bis(hydroxymethyl)harnstoff, Bis(hydroxyethyl)harnstoff; Bis(hydroxypropyl)harnstoff; N,N'-Dihydroxymethylharnstoff; N,N'-Dihydroxyethylharnstoff; N,N'-Dihydroxypropylharnstoff; N,N,N'-Trihydroxyethylharnstoff; Tetra(hydroxymethyl)harnstoff; Tetra(hydroxyethyl)harnstoff; Tetra(hydroxypropyl)harnstoff; N-Methyl-, N'-hydroxyethylharnstoff; N-Ethyl-N'-hydroxyethylharnstoff; N-Hydroxypropyl-N'-hydroxyethylharnstoff und N,N'-Dimethyl-N-hydroxyethylharnstoff oder eine Mischung davon.

9. Verwendung nach einem der Ansprüche 1 bis 8, wobei die Zusammensetzung ferner umfasst Eukalyptusöl, Lavendelöl, N,N-Diethyl-meta-toluamid (DEET), Octopirox (Pirocton), Zinkpyrithion, Benzethoniumchlorid, Benzalkoniumchlorid, Chloroxylenol, Triclosan, Cetylpyridiniumchlorid, Silberoxid, Silbernitrat, Silbersulfat, Silberphosphat, Silbercarbonat, Silberacetat, Silberbenzoat oder eine Mischung davon.

10. Verwendung nach einem der Ansprüche 1 bis 9, wobei die Zusammensetzung mindestens 82% Wasser umfasst.

11. Verwendung nach einem der Ansprüche 1 bis 10, wobei die Zusammensetzung 85 bis 99,5 Gew.-% Wasser und weniger als 5 Gew.-% Ethanol umfasst.

12. Verwendung nach einem der Ansprüche 1 bis 11, wobei die Zusammensetzung ferner Glycerin umfasst und eine Viskosität unter 30 Pa.s (30.000 cps) aufweist.

13. Verwendung nach einem der Ansprüche 1 bis 12, wobei die Zusammensetzung ferner ein Anti-Mücken-Mittel, Octopirox, Zinkpyrithion, Chloroxylenol, Triclosan, Cetylpyridiniumchlorid oder eine Silberverbindung, die ein Silberoxid, -nitrat, -sulfat, -phosphat, -carbonat, -acetat, -benzoat oder eine Mischung davon ist, umfasst.

14. Zusammensetzung, umfassend:
a) mindestens 80 Gewichtsprozent Wasser; und
b) ein kationisches Tensid,
wobei die Zusammensetzung in der Lage ist, innerhalb von weniger als 3 Minuten nach topischer Anwendung eine bakterielle Log-Abtötung von mindestens 2 und eine virale Log-Inaktivierung von mindestens 2 zu erzielen, und wobei die Zusammensetzung ferner weniger als 10 Gew.-% Ethanol aufweist und wenn die Gesamtmenge an Tensid in der Zusammensetzung mehr als 4,5 Gew.-% beträgt und das kationische Tensid in der Zusammensetzung eine Kombination aus sowohl Cetrimoniumchlorid als auch Benzalkoniumchlorid umfasst, Benzalkoniumchlorid mindestens 16,5 Gew.-% der Kombination ausmacht; wobei die Zusammensetzung weniger als 0,3 Gew.-% Benzalkoniumchlorid und/oder Benzethoniumchlorid aufweist; wobei das kationische Tensid 95 bis 100 Gew.-% Trimoniumverbindung, Dimoniumverbindung oder beides aufweist; wobei die Zusammensetzung 0,0 Gew.-% aliphatischen Alkohol umfasst, ausgenommen Ethanol und nicht umfassend Diole und Polyole; wobei die Trimoniumverbindung eine Alkyl-Trimoniumverbindung ist, umfassend Cetrimoniumchlorid, Cetrimoniumbromid, Mytrimoniumchlorid, Mytrimoniumbromid, Behentrimoniummethosulfat, Cocotrimoniummethosulfat, Behentrimoniumchlorid, Behentrimoniumbromid, Steartrimoniumchlorid, Steartrimoniumbromid, Laurtrimoniumchlorid, Laurtrimoniumbromid oder eine Mischung davon, und wobei die Dimoniumverbindung Distearyldimoniumchlorid, Didecyldimoniumchlorid, Dicocodimoniumchlorid, eine Mischung davon oder Benzethoniumchlorid und/oder Benzalkoniumchlorid ist, und wobei das kationische Tensid 0,08 bis 0,4 Gew.-% der Zusammensetzung ausmacht, zur Verwendung in einem Verfahren zur Verhinderung unerwünschter Hautreaktionen, wie Akneentwicklung, die durch Mikroben verursacht wird, die sich unter einer persönlichen Schutzausrüstung ansammeln, umfassend den Schritt des Auftragens der Zusammensetzung auf die Haut vor dem Anlegen der persönlichen Schutzausrüstung.

## Revendications

1. Utilisation non thérapeutique, en tant que composition de désinfection antibactérienne et antivirale, d'une composition comprenant :
a) au moins 80 % en poids d'eau ; et
b) un tensioactif cationique
dans laquelle la composition est capable d'induire une réduction logarithmique des bactéries d'au moins 2 et une inactivation logarithmique virale d'au moins 2 en moins de 3 minutes après une application topique et en outre dans laquelle la composition comprend moins de 10 % en poids d'éthanol et lorsque le tensioactif total dans la composition est supérieur à 4,5 % en poids et que le tensioactif cationique dans la composition comprend une combinaison à la fois de chlorure de cétrimonium et de chlorure de benzalkonium, le chlorure de benzalkonium représente au moins 16,5 % en poids de la combinaison ; dans laquelle la composition comprend moins de 0,3 % en poids de chlorure de benzalkonium et/ou de chlorure de benzéthonium ; dans laquelle le tensioactif cationique représente de 95 à 100 % en poids d'un composé trimonium, d'un composé dimonium ou des deux ; dans laquelle la composition comprend 0,0 % en poids d'alcool aliphatique à l'exclusion de l'éthanol et ne comprenant de diols et de polyols ; dans laquelle le composé trimonium est un composé alkyltrimonium comprenant le chlorure de cétrimonium, le bromure de cétrimonium, le chlorure de mytrimonium, le bromure de mytrimonium, le méthosulfate de béhentrimonium, le méthosulfate de cocotrimonium, le chlorure de béhentrimonium, le bromure de béhentrimonium, le chlorure de stéartrimonium, le bromure de stéartrimonium, le chlorure de laurtrimonium, le bromure de laurtrimonium ou un mélange de ceux-ci, et dans laquelle le composé dimonium est le chlorure de distéaryldimonium, le chlorure de didécyldimonium, le chlorure de dicocodimonium, un mélange de ceux-ci ou le chlorure de benzéthonium et/ou le chlorure de benzalkonium, et dans laquelle le tensioactif cationique représente de 0,08 à 0,4 % en poids de la composition.

2. Utilisation selon la revendication 1 dans laquelle le tensioactif cationique comprend le chlorure de cétrimonium, le bromure de cétrimonium ou un mélange de ceux-ci.

3. Utilisation selon la revendication 1 ou 2 dans laquelle le tensioactif cationique est le chlorure de cétrimonium.

4. Utilisation selon l'une quelconque des revendications 1 à 3 dans laquelle la composition comprend en outre un tensioactif non ionique.

5. Utilisation selon l'une quelconque des revendications 1 à 4 dans laquelle la composition comprend en outre un polyol et le polyol est le sorbitol, le glycérol, le mannitol, le xylitol, le maltitol, le dipropylène glycol, le polypropylène glycol, le polyéthylène glycol, l'hydroxypropylsorbitol, l'hexylène glycol, le 1,3-butylène glycol, le 1,2-octanediol, le 1,2-hexanediol, l'isoprène glycol, le 1,2,6-hexanetriol, le glycérol éthoxylé, le glycérol propoxylé ou un mélange de ceux-ci.

6. Utilisation selon l'une quelconque des revendications 1 à 5 dans laquelle la composition comprend en outre des acides gras oméga-3 solubles, des acides gras oméga-6, du climbazole, du magnolol, de l'honokiol, du farnésol, de l'acide ursolique, de l'acide myristique, du géranylgéraniol, de l'oléylbétaïne, de la cocoylhydroxyéthylimidazoline, de l'hexanoylsphingosine, de l'acide 12-hydroxystéarique, de l'acide pétrosélinique, de l'acide linoléique conjugué, de l'acide stéarique, de l'acide palmitique, de l'acide laurique, du terpinéol, des composants essentiels du thymol, l'agent de dissolution choisi parmi le limonène, le pinène, le camphène, le cymène, le citronellol, le citronellal, le géraniol, le nérol, le linalol, le rhodinol, le bornéol, l'isobornéol, la menthone, le camphre, le safrole, l'isosafrole, l'eugénol, l'isoeugénol, l'huile d'arbre à thé, l'huile d'eucalyptus, l'huile de menthe poivrée, l'huile de neem, l'huile de citronnelle, l' huile d'orange, l'huile de bergamote ou un mélange de ceux-ci.

7. Utilisation selon l'une quelconque des revendications 1 à 6 dans laquelle la composition comprend en outre de l'arginine, de la valine, de l'histidine, de la vitamine B₂, du niacinamide (vitamine B₃), de la vitamine B₅, de la vitamine B₆, de la vitamine C, du tétraisopalmitate d'ascorbyle, du phosphate d'ascorbyle de magnésium, du glycoside d'ascorbyle, de l'acide hyaluronique et des sels de celui-ci, du 4-éthylrésorcinol, de la sauge, de l'aloe vera, du thé vert, de la canne à sucre, des agrumes, des pépins de raisin, du thym, de la camomille, de l'achillée millefeuille, du concombre, de la réglisse, du romarin, de l'ensulizole, du NaCl, du KCI ou un mélange de ceux-ci.

8. Utilisation selon l'une quelconque des revendications 1 à 7 dans laquelle la composition comprend en outre de l'hydroxyméthylurée, de l'hydroxyéthylurée, de l'hydroxypropylurée ; de la bis(hydroxyméthyl)urée ; de la bis(hydroxyéthyl)urée ; de la bis(hydroxypropyl)urée ; de la N,N'-dihydroxyméthylurée ; de la N,N'-dihydroxyéthylurée ; de la N,N'-dihydroxypropylurée ; de la N,N,N'-trihydroxyéthylurée ; de la tétra(hydroxyméthyl)urée ; de la tétra(hydroxyéthyl)urée ; de la tétrahydroxypropylurée ; de la N-méthyl,N'-hydroxyéthylurée ; de la N-éthyl-N'-hydroxyéthylurée ; de la N-hydroxypropyl-N'-hydroxyéthylurée et de la N,N'-diméthyl-N-hydroxyéthylurée ou un mélange de celles-ci.

9. Utilisation selon l'une quelconque des revendications 1 à 8 dans laquelle la composition comprend en outre de l'huile d'eucalyptus, de l'huile de lavande, du N,N-diéthyl-méta-toluamide (DEET), de l'octopirox (piroctone), de la pyrithione de zinc, du chlorure de benzéthonium, du chlorure de benzalkonium, du chloroxylénol, du triclosan, du chlorure de cétylpyridinium, de l'oxyde d'argent, du nitrate d'argent, du sulfate d'argent, du phosphate d'argent, du carbonate d'argent, de l'acétate d'argent, du benzoate d'argent ou un mélange de ceux-ci.

10. Utilisation selon l'une quelconque des revendications 1 à 9 dans laquelle la composition comprend au moins 82 % d'eau.

11. Utilisation selon l'une quelconque des revendications 1 à 10 dans laquelle la composition comprend de 85 % à 99,5 % en poids d'eau et moins de 5 % en poids d'éthanol.

12. Utilisation selon l'une quelconque des revendications 1 à 11 dans laquelle la composition comprend en outre du glycérol et a une viscosité inférieure à 30 Pa.s (30 000 cps).

13. Utilisation selon l'une quelconque des revendications 1 à 12 dans laquelle la composition comprend en outre un agent antimoustique, de l'octopirox, de la pyrithione de zinc, du chloroxylénol, du triclosan, du chlorure de cétylpyridinium ou un composé d'argent qui est un oxyde, un nitrate, un sulfate, un phosphate, un carbonate, un acétate, un benzoate d'argent ou un mélange de ceux-ci.

14. Composition comprenant :
a) au moins 80 % en poids d'eau ; et
b) un tensioactif cationique
dans laquelle la composition est capable d'induire une réduction logarithmique des bactéries d'au moins 2 et une inactivation logarithmique virale d'au moins 2 en moins de 3 minutes après une application topique et en outre dans laquelle la composition comprend moins de 10 % en poids d'éthanol et lorsque le tensioactif total dans la composition est supérieur à 4,5 % en poids et que le tensioactif cationique dans la composition comprend une combinaison à la fois de chlorure de cétrimonium et de chlorure de benzalkonium, le chlorure de benzalkonium représente au moins 16,5 % en poids de la combinaison ; dans laquelle la composition comprend moins de 0,3 % en poids de chlorure de benzalkonium et/ou de chlorure de benzéthonium ; dans laquelle le tensioactif cationique représente de 95 à 100 % en poids d'un composé trimonium, d'un composé dimonium ou des deux ; dans laquelle la composition comprend 0,0 % en poids d'alcool aliphatique à l'exclusion de l'éthanol et ne comprenant de diols et de polyols ; dans laquelle le composé trimonium est un composé alkyltrimonium comprenant le chlorure de cétrimonium, le bromure de cétrimonium, le chlorure de mytrimonium, le bromure de mytrimonium, le méthosulfate de béhentrimonium, le méthosulfate de cocotrimonium, le chlorure de béhentrimonium, le bromure de béhentrimonium, le chlorure de stéartrimonium, le bromure de stéartrimonium, le chlorure de laurtrimonium, le bromure de laurtrimonium ou un mélange de ceux-ci, et dans laquelle le composé dimonium est le chlorure de distéaryldimonium, le chlorure de didécyldimonium, le chlorure de dicocodimonium, un mélange de ceux-ci ou le chlorure de benzéthonium et/ou le chlorure de benzalkonium, et dans laquelle le tensioactif cationique représente de 0,08 à 0,4 % en poids de la composition, pour une utilisation dans un procédé de prévention de réactions cutanées indésirables, telles que le développement de l'acné, causées par des microbes qui s'accumulent sous les équipements de protection individuelle comprenant l'étape d'application de la composition sur la peau avant la mise en place de l'équipement de protection individuelle.
